# EUROPEAN PATENT APPLICATION

(11) **EP 4 818 166 A1**
(43) Date of publication of application: **07.10.2026**
(21) Application number: 25168524.4
(22) Date of filing: 04.04.2025
(51) Int. Cl.: C07D 413/12, A01N 43/80

(54) **PROCESS FOR THE PREPARATION OF PYROXASULFONE**

(71) Applicant: Adama Agan Ltd., 7710201 Ashdod (IL)
(72) Inventor: ASHUSH, Natali, 4284200 Bat Hefer (IL); COHEN, Yair, 7685925 Mazkeret Batya (IL)
(74) Representative: Modiano, Gabriella Diana

(57) **Abstract**

The present application relates to a process for the preparation of pyroxasulfone comprising reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst, a cationic surfactant and water.

## Description

### FIELD OF THE INVENTION

The present invention relates to the field of synthesis of organic compounds, more specifically to a process for the preparation of pyroxasulfone.

### BACKGROUND PRIOR ART

US2023012374 discloses the preparation of pyroxasulfone (3-[[5-(difluoromethoxy)-1-methyl-3-(trifluoromethyl)pyrazol-4-yl]methylsulfonyl]-5,5-dimethyl-4H-1,2-oxazole) by oxidizing 3-[(5-difluoromethoxy-1-methyl-3-trifluoromethylpyrazol-4-yl)methylthio]-4,5-dihydro-5,5-dimethylisoxazole in the presence of a metal catalyst and hydrogen peroxide. The yields of the reaction are typically lowered by incomplete oxidation of the thio group to the corresponding sulfoxide. Different organic solvents are tested in US2023012374, for example, butyl acetate, isopropyl acetate, ethyl acetate, methanol, butanol, ethanol, acetonitrile, DMF, or NMP, each solvent and solvent amount having a different effect on the amount pyroxasulfone formed and the resulting amounts of the sulfoxide intermediate impurity. According to the tested organic solvents in US2023012374, the amount of organic solvent ranges from 0.4 to 1.8 liters of organic solvent per mole of 3-[(5-difluoromethoxy-1-methyl-3-trifluoromethylpyrazol-4-yl)methylthio]-4,5-dihydro-5,5-dimethylisoxazole.

### SUMMARY OF THE INVENTION

The present invention provides an alternative to the processes for the preparation of pyroxasulfone, wherein no organic solvent or a negligible amount of organic solvent is present during the oxidation reaction for the preparation of pyroxasulfone. According to an embodiment of this invention, conducting the oxidation in the presence of a metal catalyst and a cationic surfactant avoids or significantly reduces the use of organic solvents during the oxidation reaction, as comparison with the prior art, while the pyroxasulfone yield is excellent, and the amount of sulfoxide impurity is acceptable. The reduction in the amount of organic solvent means a reduction in the cost of the process, as well as the reaction waste.

Thus, the present application is directed to a process for the preparation of pyroxasulfone (3-[[5-(difluoromethoxy)-1-methyl-3-(trifluoromethyl)pyrazol-4-yl]methylsulfonyl]-5,5-dimethyl-4H-1,2-oxazole) comprising reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst selected from the groups 5 to 6 of the periodic table, at least one cationic surfactant and water.

The reaction can be summarized in the following equation:

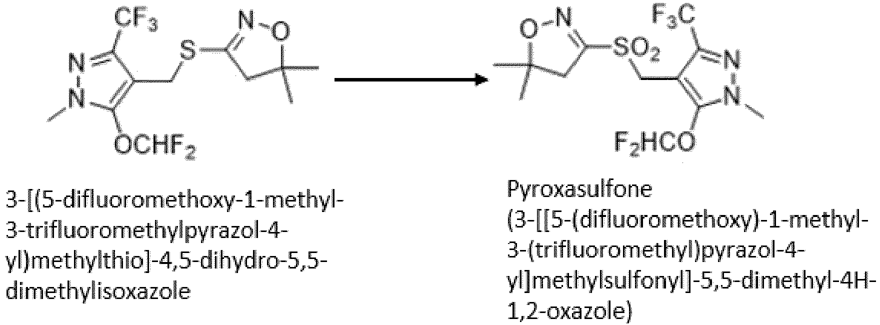

### DETAILED DESCRIPTION OF THE INVENTION

### Definitions

Embodiments of the present invention are discussed in detail below. In describing embodiments, specific terminology is employed for the sake of clarity. However, the invention is not intended to be limited to the specific terminology so selected. While specific exemplary embodiments are discussed, it should be understood that this is done for illustration purposes only. While a number of embodiments and features are described herein, it is to be understood that the various features of the invention and aspects of embodiments, even if described separately, may be combined unless mutually exclusive or contrary to the specific description. All references cited herein are incorporated by reference as if each had been individually incorporated.

As used herein, the transitional term "comprising" or "that comprises", which is synonymous with "including," or "containing," is inclusive or open-ended and does not exclude additional, un-recited elements or method steps. However, in each recitation of "comprising" herein, it is intended that the term also encompass, as alternative embodiments, the phrases "consisting essentially of" and "consisting of", where "consisting of" excludes any element or step not specified and "consisting essentially of" permits the inclusion of additional un-recited elements or steps that do not materially affect the essential or basic and novel characteristics of the composition or method under consideration.

Prior to setting forth the present subject matter in detail, it may be helpful to provide definitions of certain terms to be used herein. Unless defined otherwise, all technical and scientific terms used herein have the same meaning as is commonly understood by one of skill in the art to which this subject matter pertains.

The term "C₁-C₂₄ alkyl" means a linear or branched saturated hydrocarbon chain radical having no multiple bonds and having from one to twenty-four carbon atoms, and which is attached to the rest of the molecule by a single bond. Suitable groups include, but are not limited to, alkyl groups such as methyl, ethyl, propyl (e.g. *n*-propyl or *iso*-propyl), butyl (e.g. *n*-butyl, *t*-butyl, *sec*-butyl), pentyl (e.g. 1-methylpentyl, 3-methylpentyl, *n*-pentyl), hexyl, octyl, dodecyl, hexadecyl or octadecyl.

The term "a" or "an" as used herein includes the singular and the plural, unless specifically stated otherwise. Therefore, the terms "a," "an" or "at least one" can be used interchangeably in this application.

For purposes of better understanding the present teachings and in no way limiting the scope of the teachings, unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques. In this regard, used of the term "about" herein specifically includes ±10% from the indicated values in the range. In addition, the endpoints of all ranges directed to the same component or property herein are inclusive of the endpoints, are independently combinable, and include all intermediate points and ranges. Similarly, the ranges and amounts for each element of the technology described herein can be used together with ranges or amounts for any of the other elements.

### Process for the preparation of pyroxasulfone

In a first aspect, the present disclosure relates to a process for the preparation of pyroxasulfone comprising reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst selected from the groups 5 to 6 of the periodic table, at least one cationic surfactant and water, wherein the total amount of organic solvents in the reaction is less than 0.05 liters of organic solvents per mole of 3-[(5-difluoromethoxy-1-methyl-3-trifluoromethylpyrazol-4-yl)methylthio]-4,5-dihydro-5,5-dimethylisoxazole.

This process provides an alternative to the existing processes for the preparation of pyroxasulfone, and particularly, it provides a process wherein no organic solvent, or a negligible amount of organic solvent, is present during the oxidation reaction, while the yield of pyroxasulfone is excellent, and the amount of sulfoxide impurity is less than 5%, less than 4%, in comparison with the processes where a significant proportion of organic solvent is required during the oxidation reaction for the preparation of pyroxasulfone.

The at least one cationic surfactant may be selected from the group consisting of (C₈-C₂₄)alkyltrimethylammonium halide, (C₈-C₂₄)dialkyldimethylammonium halide and (C₈-C₂₄)trialkylmethylammonium halide, or mixtures thereof.

The at least one cationic surfactant may be selected from the group consisting of (C₁₀-C₂₀)alkyltrimethylammonium halide and (C₁₀-C₂₀)dialkyldimethylammonium halide, or mixtures thereof.

The at least one cationic surfactant may be selected from a (C₁₀-C₂₀)alkyltrimethylammonium halide.

The at least one cationic surfactant may be selected from cetrimonium bromide or chloride, or mixtures thereof.

The amount of the at least one cationic surfactant may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole.

The amount of the at least one cationic surfactant may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one cationic surfactant may be selected from the group consisting of (C₈-C₂₄)alkyltrimethylammonium halide, (C₈-C₂₄)dialkyldimethylammonium halide and (C₈-C₂₄)trialkylmethylammonium halide, or mixtures thereof. The amount of the at least one cationic surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one cationic surfactant may be selected from the group consisting of (C₁₀-C₂₄)alkyltrimethylammonium halide, (C₁₀-C₂₄)dialkyldimethylammonium halide, or mixtures thereof. The amount of the at least one cationic surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one cationic surfactant may be a (C₁₀-C₂₀)alkyltrimethylammonium halide. The amount of the at least one cationic surfactant may range from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one cationic surfactant may be selected from cetrimonium bromide or chloride.

The amount of water may range from 0.1 to 3, from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole. The amount of water in the process of the present invention includes water added as solvent in the reaction vessel and water included in the aqueous hydrogen peroxide solution.

The amount of water may range from 0.1 to 3, from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole and the amount of the at least one cationic surfactant may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole. The amount of water may range from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole and the amount of the at least one cationic surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole. The amount of water may range from 0.3 to 1 liters per mole of 3-[(5-difluoromethoxy-1-methyl-3-trifluoromethylpyrazol-4-yl)methylthio]-4,5-dihydro-5,5-dimethylisoxazole and the amount of the at least one cationic surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole.

The amount of water may range from 0.1 to 3, from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one cationic surfactant may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one cationic surfactant may be selected from the group consisting of (C₈-C₂₄)alkyltrimethylammonium halide, (C₈-C₂₄)dialkyldimethylammonium halide and (C₈-C₂₄)trialkylmethylammonium halide, or mixtures thereof. The amount of water may range from 0.2 to 2, from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one cationic surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one cationic surfactant may be selected from the group consisting of (C₁₀-C₂₄)alkyltrimethylammonium halide, (C₁₀-C₂₄)dialkyldimethylammonium halide, or mixtures thereof. The amount of water may range from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one cationic surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one cationic surfactant may be a (C₁₀-C₂₀)alkyltrimethylammonium halide. The amount of water may range from 0.3 to 1 liters per mole of 3-[(5-difluoromethoxy-1-methyl-3-trifluoromethylpyrazol-4-yl)methylthio]-4,5-dihydro-5,5-dimethylisoxazole, the amount of the at least one cationic surfactant may range from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one cationic surfactant may be selected from cetrimonium bromide or chloride.

The total amount of organic solvents in the reaction may be 0 liters of organic solvents per mole of 3-[(5-difluoromethoxy-1-methyl-3-trifluoromethylpyrazol-4-yl)methylthio]-4,5-dihydro-5,5-dimethylisoxazole, i.e., no organic solvent in the reaction of the process of the invention.

The hydrogen peroxide in the process of the invention may be a 10 to 70 wt %, 20 to 65%, 25% to 60% aqueous hydrogen peroxide solution.

The amount of hydrogen peroxide may range from 2 to 9, from 2 to 7, from 2 to 5 moles per mole of 3-[(5-difluoromethoxy-1-methyl-3-trifluoromethylpyrazol-4-yl)methylthio]-4,5-dihydro-5,5-dimethylisoxazole.

The amount of hydrogen peroxide may range from 2 to 9, from 2 to 7, from 2 to 5 moles per mole of 3-[(5-difluoromethoxy-1-methyl-3-trifluoromethylpyrazol-4-yl)methylthio]-4,5-dihydro-5,5-dimethylisoxazole and the hydrogen peroxide may be a 10 to 70 wt %, 20 to 65%, 25% to 60% aqueous hydrogen peroxide solution. The amount of hydrogen peroxide may range from 2 to 7, from 2 to 5 moles per mole of 3-[(5-difluoromethoxy-1-methyl-3-trifluoromethylpyrazol-4-yl)methylthio]-4,5-dihydro-5,5-dimethylisoxazole and the hydrogen peroxide may be a 20 to 65%, 25% to 60% aqueous hydrogen peroxide solution. The amount of hydrogen peroxide may range from 2 to 5 moles per mole of 3-[(5-difluoromethoxy-1-methyl-3-trifluoromethylpyrazol-4-yl)methylthio]-4,5-dihydro-5,5-dimethylisoxazole and the hydrogen peroxide may be a 25% to 60% aqueous hydrogen peroxide solution.

The metal catalyst selected from the groups 5 to 6 of the periodic table may be selected from a tungsten catalyst, a molybdenum catalyst and a niobium catalyst. The tungsten catalyst may be selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or a mixture thereof. The tungsten catalyst may be selected from the group of tungstic acid, alkali or alkaline earth salt of tungstic acid, metal tungsten, tungsten oxide, tungsten carbide, or a mixture thereof. The molybdenum catalyst may be selected from the group of molybdic acid, molybdic acid salt, metal molybdenum, molybdenum oxide, molybdenum carbide, molybdenum chloride, or a mixture thereof. The niobium catalyst may be selected from the group of niobic acid, niobic acid salt, metal niobium, niobium oxide, niobium carbide, niobium chloride, or a mixture thereof. The metal catalyst selected from the groups 5 to 6 of the periodic table may be sodium tungstate dihydrate or ammonium molybdate tetrahydrate.

The amount of metal catalyst selected from the groups 5 to 6 of the periodic table may range from 0.001 to 0.1, from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole.

The amount of metal catalyst may range from 0.001 to 0.1, from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be selected from a tungsten catalyst, a molybdenum catalyst and a niobium catalyst. The amount of metal catalyst may range from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or mixtures thereof. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole and the metal catalyst selected from the groups 5 to 6 of the periodic table may be sodium tungstate dihydrate or ammonium molybdate tetrahydrate.

The amount of metal catalyst selected from the groups 5 to 6 of the periodic table may range from 0.001 to 0.1, from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole, and the amount of the at least one cationic surfactant may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole. The amount of metal catalyst selected from the groups 5 to 6 of the periodic table may range from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole, and the amount of the at least one cationic surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole. The amount of metal catalyst selected from the groups 5 to 6 of the periodic table may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole, and the amount of the at least one cationic surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole.

The amount of metal catalyst may range from 0.001 to 0.1, from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be selected from a tungsten catalyst, a molybdenum catalyst and a niobium catalyst, the amount of the at least one cationic surfactant may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one cationic surfactant may be selected from the group consisting of (C₈-C₂₄)alkyltrimethylammonium halide, (C₈-C₂₄)dialkyldimethylammonium halide and (C₈-C₂₄)trialkylmethylammonium halide, or mixtures thereof. The amount of metal catalyst may range from 0.005 to 0.1, from 0.01 to 0.05 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, the amount of the at least one cationic surfactant may range from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one cationic surfactant may be selected from the group consisting of (C₁₀-C₂₄)alkyltrimethylammonium halide, (C₁₀-C₂₄)dialkyldimethylammonium halide, or mixtures thereof. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or mixtures thereof, the amount of the at least one cationic surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one cationic surfactant may be a (C₁₀-C₂₀)alkyltrimethylammonium halide. The amount of metal catalyst may range from 0.01 to 0.05 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be sodium tungstate dihydrate or ammonium molybdate tetrahydrate, the amount of the at least one cationic surfactant may range from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one cationic surfactant may be selected from cetrimonium bromide or chloride.

The temperature of the reaction in the process of the present invention may range from 30°C to 120°C, from 40°C to 110°C, from 50°C to 100°C.

The reaction in the process of the present invention may be performed in a time that ranges from 1 hour to 24 hours, from 2 hours to 16 hours, from 3 hours to 12 hours.

The reaction in the process of the present invention may be performed in a time that ranges from 1 hour to 24 hours, from 2 hours to 16 hours, from 3 hours to 12 hours and the temperature of the reaction may range from 30°C to 120°C, from 40°C to 110°C, from 50°C to 100°C. The reaction in the process of the present invention may be performed in a time that ranges from 2 hours to 16 hours, from 3 hours to 12 hours and the temperature of the reaction may range from 40°C to 110°C, from 50°C to 100°C. The reaction in the process of the present invention may be performed in a time that ranges from 3 hours to 12 hours and the temperature of the reaction may range from 50°C to 100°C.

The process of the present invention may further comprise a phase transfer catalyst. The process of the present invention may further comprise a phase transfer catalyst selected from the group of tetrabutylammonium chloride, tetrabutylammonium bromide, tetrabutylammonium hydrogen sulfate, tetraoctyl ammonium bromide, tetraoctyl ammonium bromide, methyltrialkyl ammonium chloride (Adogen 464), tetradecyltrimethyl ammonium bromide, methyltrioctyl ammonium chloride (Aliquat 336), didecyldimethyl ammonium chloride, benzyltrimethyl ammonium chloride, benzyltriethyl ammonium chloride, octyltrimethyl ammonium chloride, octyltrimethyl ammonium bromide, trioctylmethyl ammonium chloride, trioctylmethyl ammonium bromide, tetrabutyl phosphonium bromide, tetraoctyl phosphonium bromide or tetraphenyl phosphonium bromide.

The amount of the further phase transfer catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole.

The process of the present invention may further comprise an acid catalyst. The process of the present invention may further comprise an acid catalyst selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate.

The amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole.

The amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate. The amount of the further acid catalyst may range from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid. The amount of the further acid catalyst may range from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, p-toluenesulfonic acid, phosphoric acid.

The metal catalyst selected from the groups 5 to 6 of the periodic table may be selected from a tungsten catalyst, a molybdenum catalyst and a niobium catalyst, the amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate. The metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, the amount of the further acid catalyst may range from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid. The metal catalyst selected from the groups 5 to 6 of the periodic table may be sodium tungstate dihydrate or ammonium molybdate tetrahydrate, the amount of the further acid catalyst may range from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, p-toluenesulfonic acid, phosphoric acid.

The amount of metal catalyst may range from 0.001 to 0.1, from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be selected from a tungsten catalyst, a molybdenum catalyst and a niobium catalyst, the amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate. The amount of metal catalyst may range from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, the amount of the further acid catalyst may range from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, trifluoromethanesulfonic acid, benzenesulfonic acid, p-toluenesulfonic acid, phosphoric acid. The amount of metal catalyst may range from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be sodium tungstate dihydrate or ammonium molybdate tetrahydrate, the amount of the further acid catalyst may range from 0.01 to 0.07 moles per mole of 3-[(5-difluoromethoxy-1-methyl-3-trifluoromethylpyrazol-4-yl)methylthio]-4,5-dihydro-5,5-dimethylisoxazole, and the acid catalyst may be selected from the group of hydrochloric acid, sulfuric acid, methanesulfonic acid, p-toluenesulfonic acid, phosphoric acid.

The amount of metal catalyst may range from 0.001 to 0.1, from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be selected from a tungsten catalyst, a molybdenum catalyst and a niobium catalyst, the amount of the further acid catalyst may range from 0 to 0.1, from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, the acid catalyst may be selected from the group of hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate, the amount of the at least one cationic surfactant may range from 0.1% to 16%, from 0.5% to 14%, from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one cationic surfactant may be selected from the group consisting of (C₈-C₂₄)alkyltrimethylammonium halide, (C₈-C₂₄)dialkyldimethylammonium halide and (C₈-C₂₄)trialkylmethylammonium halide, or mixtures thereof. The amount of metal catalyst may range from 0.01 to 0.1, from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be a tungsten catalyst selected from the group of tungstic acid, tungstic acid salt, metal tungsten, tungsten oxide, tungsten carbide, tungsten chloride, tungsten bromide, tungsten sulfide, phosphotungstic acid or a salt thereof, silicotungstic acid or a salt thereof; or a molybdenum catalyst selected from the group of molybdic acid, molybdic acid salt, metal molybdenum, molybdenum oxide, molybdenum carbide, molybdenum chloride; or a mixture thereof, the amount of the further acid catalyst may range from 0.001 to 0.1, from 0.01 to 0.07 moles per mole of 3-[(5-difluoromethoxy-1-methyl-3-trifluoromethylpyrazol-4-yl)methylthio]-4,5-dihydro-5,5-dimethylisoxazole, the acid catalyst may be selected from the group of hydrochloric acid, sulfuric acid, methanesulfonic acid, trifluoromethanesulfonic acid, benzenesulfonic acid, p-toluenesulfonic acid, phosphoric acid, the amount of the at least one cationic surfactant may range from 0.7% to 12%, from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one cationic surfactant may be a (C₁₀-C₂₀)alkyltrimethylammonium halide. The amount of metal catalyst may range from 0.03 to 0.07 moles per mole of 3-[(5-difluoromethoxy-1-methyl-3-trifluoromethylpyrazol-4-yl)methylthio]-4,5-dihydro-5,5-dimethylisoxazole, the metal catalyst selected from the groups 5 to 6 of the periodic table may be sodium tungstate dihydrate or ammonium molybdate tetrahydrate, the amount of the further acid catalyst may range from 0.01 to 0.07 moles per mole of 3-[(5-difluoromethoxy-1-methyl-3-trifluoromethylpyrazol-4-yl)methylthio]-4,5-dihydro-5,5-dimethylisoxazole, the acid catalyst may be selected from the group of hydrochloric acid, sulfuric acid, methanesulfonic acid, p-toluenesulfonic acid, phosphoric acid, the amount of the at least one cationic surfactant may range from 1% to 10% by weight of surfactant vs weight of 3-[(5-difluoromethoxy-1-methyl-3-trifluoromethylpyrazol-4-yl)methylthio]-4,5-dihydro-5,5-dimethylisoxazole, and the at least one cationic surfactant may be selected from cetrimonium bromide or chloride.

The process of the present invention may comprise the following steps: i) addition of 3-[(5-difluoromethoxy-1-methyl-3-trifluoromethylpyrazol-4-yl)methylthio]-4,5-dihydro-5,5-dimethylisoxazole, addition of the at least one cationic surfactant, and optional addition of the metal catalyst, water and organic solvent to the reaction vessel; ii) addition of the aqueous hydrogen peroxide solution; iii) optional addition of the metal catalyst, water and organic solvent; iv) isolation and purification of the obtained pyroxasulfone from the reaction mixture, with the proviso that the metal catalyst selected from the groups 5 to 6 of the periodic table is added at least in one of the steps i) or iii).

The metal catalyst selected from the groups 5 to 6 of the periodic table may be added to the reaction vessel before and/or after the addition of the aqueous hydrogen peroxide solution. Water as solvent may be added to the reaction vessel before and/or after the addition of the aqueous hydrogen peroxide solution, or it may not be added to the reaction vessel. The organic solvent in an amount of less than 0.05 liters of organic solvents per mole of 3-[(5-difluoromethoxy-1-methyl-3-trifluoromethylpyrazol-4-yl)methylthio]-4,5-dihydro-5,5-dimethylisoxazole, may be added to the reaction vessel before and/or after the addition of the aqueous hydrogen peroxide solution, or it may not be added to the reaction vessel.

The metal catalyst selected from the groups 5 to 6 of the periodic table may be added to the reaction vessel as solid or pre-dissolved in water or in the organic solvent, it may be added in one, two, or several portions, or it may be added in continuous way.

The aqueous hydrogen peroxide solution may be added at once, in several portions, or it may be added dropwise.

The reaction of the process of the present invention may be carried out under high-shear mixing. The under high-shear mixing may be carried out at between 2500 rpm and 30000 rpm.

Previously to the isolation and purification of the obtained pyroxasulfone from the reaction mixture, any possible unreacted hydrogen peroxide may be decomposed by treating the reaction mixture with any reducing agent known by the person skilled in the art, like for example and in a not limiting way, sodium sulfite.

The isolation, purification or removal of unreacted reactants, reagents, additives and/or impurities from pyroxasulfone may be performed by any method known by the person skilled in the art, like for example and in a not limiting way, dissolution in organic solvent, decantation, extraction, washing with aqueous or organic solvent, or crystallization in organic solvent.

A second aspect of the invention is the use of pyroxasulfone obtained according to the process hereinabove disclosed in the preparation of an agrochemical composition.

### Examples

### Example 1: Oxidation with hydrogen peroxide, sodium tungstate as catalyst, and cetrimonium bromide as surfactant

3-[(5-difluoromethoxy-1-methyl-3-trifluoromethylpyrazol-4-yl)methylthio]-4,5-dihydro-5,5-dimethylisoxazole (97.5 gr, 75 % purity) is added to a 1-liter reactor at 60 °C followed by 100 ml of water and stirring is started. The surfactant cetrimonium bromide is added in an amount of 2.74% by weight of 3-[(5-difluoromethoxy-1-methyl-3-trifluoromethylpyrazol-4-yl)methylthio]-4,5-dihydro-5,5-dimethylisoxazole. A solution of catalyst is prepared as follows: Sodium tungstate dihydrate (1.38 gr) is dissolved in 3.62 ml of water. Half of the catalyst solution (2.5 gr) is added to the reactor. Then, the reaction is fed with hydrogen peroxide (30%, 4.2 eq., 100 gr) dropwise for 2 hrs. At the beginning of the addition a slight exotherm is observed, and after the addition of the 10% of the hydrogen peroxide, the temperature is increased to 95 °C. After the addition of hydrogen peroxide, the second half of the catalyst solution is added. After aging of additional 4 hours the sulfoxide impurity, 3-[(5-difluoromethoxy-1-methyl-3-trifluoromethylpyrazol-4-yl)methylsulfoxide]-4,5-dihydro-5,5-dimethylisoxazole, relative HPLC area percentage is 2.1%, and pyroxasulfone relative HPLC area percentage is 97.9%. The aqueous phase is removed by decantation from the reactor, and the obtained pellets of pyroxasulfone were dissolved in organic solvent for the work-up.

## Claims

1. A process for the preparation of pyroxasulfone comprising reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst selected from the groups 5 to 6 of the periodic table, at least one cationic surfactant and water, wherein the total amount of organic solvents in the reaction is less than 0.05 liters of organic solvents per mole of 3-[(5-difluoromethoxy-1-methyl-3-trifluoromethylpyrazol-4-yl)methylthio]-4,5-dihydro-5,5-dimethylisoxazole.

2. The process according to claim 1, wherein the cationic surfactant is selected from the group consisting of (C₈-C₂₄)alkyltrimethylammonium halide, (C₈-C₂₄)dialkyldimethylammonium halide, (C₈-C₂₄)trialkylmethylammonium halide, or mixtures thereof.

3. The process according to claim 2, wherein the cationic surfactant is selected from the group consisting of (C₁₀-C₂₀)alkyltrimethylammonium halide, (C₁₀-C₂₀)dialkyldimethylammonium halide, or mixtures thereof.

4. The process according to claim 3, wherein the cationic surfactant is a (C₁₀-C₂₀)alkyltrimethylammonium halide.

5. The process according to claim 4, wherein the cationic surfactant is selected from cetrimonium bromide or chloride.

6. The process according to any of the previous claims, wherein the amount of the at least one cationic surfactant ranges from 0.1% to 16% by weight of surfactant vs weight of 3-[(5-difluoromethoxy-1-methyl-3-trifluoromethylpyrazol-4-yl)methylthio]-4,5-dihydro-5,5-dimethylisoxazole.

7. The process according to any of the previous claims, wherein it does not comprise any organic solvent in the reaction.

8. The process according to any of the previous claims, wherein the amount of water ranges from 0.1 to 3 liters per mole of 3-[(5-difluoromethoxy-1-methyl-3-trifluoromethylpyrazol-4-yl)methylthio]-4,5-dihydro-5,5-dimethylisoxazole.

9. The process according to any of the previous claims, wherein the hydrogen peroxide is a 10 to 70 wt percent aqueous hydrogen peroxide solution.

10. The process according to any of the previous claims, wherein the metal catalyst selected from the groups 5 to 6 of the periodic table is selected from a tungsten catalyst, a molybdenum catalyst and a niobium catalyst.

11. The process according to claim 10, wherein the metal catalyst selected from the groups 5 to 6 of the periodic table is a tungsten catalyst.

12. The process according to claim 11, wherein the metal catalyst selected from the groups 5 to 6 of the periodic table is selected from the group consisting of tungstic acid, a tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or a mixture of them.

13. The process according to claim 10, wherein the metal catalyst selected from the groups 5 to 6 of the periodic table is sodium tungstate dihydrate or ammonium molybdate tetrahydrate.

14. The process according to any of the previous claims, wherein the temperature of the reaction is between 30°C and 120°C.

15. The process according to any of the previous claims, wherein the reaction is under high-shear mixing.
